# EUROPEAN PATENT APPLICATION

(11) **EP 3 679 856 A1**
(43) Date of publication of application: **15.07.2020**
(21) Application number: 18854905.9
(22) Date of filing: 07.09.2018
(51) Int. Cl.: A61B 5/00

(54) **CHROMATOGRAPHIC STRIP COMPRISING MULTIPLE TEST LINES, DIAGNOSTIC KIT COMPRISING SAME, AND QUALITATIVE, SEMI-QUANTITATIVE OR QUANTITATIVE ANALYSIS METHOD COMPRISING MULTIPLE COMPETITIVE REACTION MEASUREMENT STEPS**

(30) Priority: 07.09.2017 KR 20170114346
(71) Applicant: Proteometech Inc., Seoul 03722 (KR)
(72) Inventor: LIM, Kook Jin, Seoul 06291 (KR); KIM, Tae Hyoung, Seoul 08847 (KR); PARK, Dong Suk, Seongnam-si Gyeonggi-do 13586 (KR); SHIN, Seok Kyo, Namyangju-si Gyeonggi-do 12253 (KR); KIM, Mi Jeoung, Seoul 02638 (KR)
(74) Representative: J A Kemp LLP
(86) International application number: PCT/KR2018/010479
(87) International publication number: WO 2019/050321

(57) **Abstract**

The present invention relates to: a chromatographic strip comprising a plurality of test lines for a competitive assay; a diagnostic kit comprising the chromatographic strip; and a method for qualitatively or quantitatively analyzing a target material within a specimen by using the same. By using the chromatographic strip of the present invention, qualitative analysis and highly reliable quantitative analysis can be rapidly and conveniently carried out with the naked eyes or a sensor even for a target material, which has one ligand binding position or is small. Furthermore, the present invention has an effect of improving the concentration measurement range of an analyte by the mutual supplementation of test lines and control lines.

## Description

### [Technical Field]

The present invention relates to a chromatographic strip having multiple test lines, a diagnostic kit including the chromatographic strip, and a method of qualitatively and/or quantitatively determining a target material in a sample using multiple competition reactions. The chromatographic analysis device of the present invention can be used for qualitative, semi-quantitative, and quantitative analyses in various fields such as the diagnosis and prediction of disease, food safety inspection, environmental analysis, compound analysis, and the like.

### [Background Art]

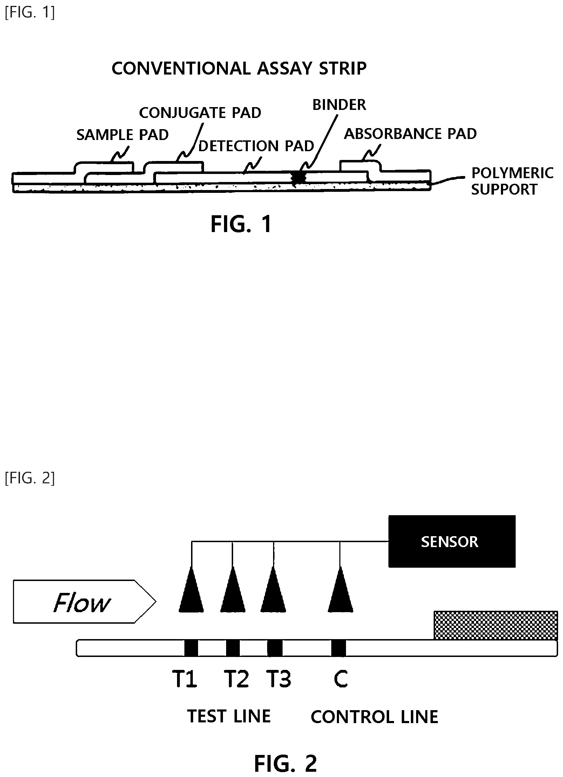

The chromatographic analysis, known as a rapid test method, is used in various fields such as medicine, agriculture, animal husbandry, food, military, and the environment by being capable of qualitatively and quantitatively determining a trace amount of a target material (i.e., analyte) in a short time using a binding reaction between the analyte and a ligand that can specifically bind to the analyte, and the application thereof includes the diagnosis or inspection of various diseases. The chromatographic analysis is generally performed using an assay strip containing a reactant capable of undergoing a change by reacting with the analyte to be detected or a device-type analyzer in which such an assay strip is mounted in a polymeric case. FIG. 1 is a cross-sectional view of a conventional assay strip used for chromatographic analysis. As shown in FIG. 1, a conventional assay strip consists of: a sample pad for accommodating a liquid sample; a conjugate pad containing a conjugate in which a labeling material (e.g., a fluorescent material or a gold particle) for generating a signal that can be detected with the naked eye or a sensor is conjugated to a ligand such as a nucleic acid, an antigen, or an antibody; a detection pad having a test line to which a binder (e.g., a nucleic acid, an antibody, or an antigen) capable of specifically binding to the analyte contained in the sample and/or to the conjugate has been immobilized and a control line through which the migration of the sample can be confirmed; and an absorbance pad for finally accommodating the liquid sample, wherein the above-described functional pads are attached on a solid support in such a manner that they are continuously arranged in the order listed above by being partially overlapped with a neighboring pad (or pads). When such an assay strip is mounted inside a polymeric case for its use, a sample inlet for loading a sample onto the sample pad is provided at an upper portion of the case, and a result display window through which a test result can be viewed is provided where the binder of the detection pad has been immobilized.

In a chromatographic analysis method using such an assay strip, when a liquid sample is loaded onto the sample pad, the liquid sample moves through the conjugate pad and the detection pad by capillary action and is finally accommodated in the absorbance pad. In one exemplary case in which an antibody (i.e., a first binding antibody) capable of binding to an antigen to be detected has been immobilized, in the form of a line (i.e., a test line), to the detection pad and another antibody (i.e., a second binding antibody) has been bound to a labeling material such as a gold particle, when a sample is allowed to migrate, a part of the sample migrating through the conjugate pad forms a complex consisting of the antigen, the gold particle, and the second binding antibody, which further migrates until it is captured by the first binding antibody immobilized in the test line to form "a complex consisting of the first binding antibody, the antigen, the gold particle, and the second binding antibody, which is immobilized to the test line". As a result, the test line appears red due to the gold particles. As described above, when a liquid sample migrates, a conjugate contained in the conjugate pad also migrates, and when the sample being analyzed contains the analyte of interest, the conjugate binds to an immobilized binder of the detection pad through the analyte (usually referred to as "a sandwich reaction").

However, in order for an analysis based on a sandwich reaction to be established, the analyte should have two or more binding sites (i.e., epitopes) for an antibody or ligand so that it can be bound to a first binding antibody as well as to a conjugate consisting of a second binding antibody and a labeling material through a sandwich reaction. In addition, an analysis based on a sandwich reaction has problems in that in the case of small-sized analytes (e.g., peptides, drugs, allergens, and the like), binding affinity may be weakened due to steric hindrance or the like, and depending on the host from which the antibody is produced or the like, specificity, sensitivity, and cross-reactivity issues may arise.

Therefore, in order to overcome the above-described problems, one alternative is to immobilize the analyte in a test line of the detection pad so that the analyte immobilized in the test line and the analyte contained in a sample compete over a binding site in a conjugate consisting of an antibody and a labeling material of the conjugate pad, in which case the signal generated from the test line weakens as the analyte concentration of the sample increases (such a competitive reaction is usually referred to as "a competition reaction"). The use of an assay strip to which the above-described principle has been applied allows the determination of the presence or absence of the analyte in a sample either by visual inspection or a sensor.

However, while a rapid kit based on the above-described competition reaction provides benefits of easy mass-production, easy operation, fast reading of results, and a low analysis cost due to not requiring an expensive, large-scale specialized analytical device, there is also a disadvantage in that the kit has a relatively low detection limit, and due to generally relying on visual inspection, produces relatively poor quantification performance. For this reason, the conventional chromatographic analysis methods mainly used for qualitative analyses have many shortcomings in use for quantitative analyses.

### [Disclosure]

### [Technical Problem]

As a result of extensive research efforts to realize a chromatographic strip capable of quantitatively and qualitatively determining an analyte which is difficult to determine using a typical chromatographic strip utilizing a sandwich reaction, the inventors of the present invention have developed a chromatographic strip distinguished from a conventional chromatographic strip, which has one test line including a material the same as or similar to the analyte and utilizes a competition reaction, by having additional test line(s) to allow multiple competition reactions to take place, and have confirmed that the chromatographic strip which allows the intensity of test line signals to be compared among the test lines or with the intensity of a control line signal can be used to quantify the analyte over a wide dynamic range of analyte concentration.

Therefore, the present invention is directed to providing a chromatographic strip which uses multiple competition reactions and thus can be used for quantitative analysis and has a high detection limit.

The present invention is also directed to providing a diagnosis kit including the chromatographic strip.

In addition, the present invention is directed to providing a method of qualitatively and quantitatively determining the analyte contained in a sample using the chromatographic strip or the diagnosis kit.

### [Technical Solution]

One aspect of the present invention provides a chromatographic strip including a conjugate pad and a detection pad. In the chromatographic strip, the conjugate pad contains a first ligand for reacting with the analyte and a labeling material capable of generating a detectable signal and binding to the first ligand, and the detection pad has two or more test lines separated from each other, wherein the first ligand and the labeling material are joined as a first conjugate before or during the migration of a sample, the conjugate can migrate to the detection pad during the migration of the sample, and in each of the two or more test lines, a material the same as or similar to the analyte has been immobilized so that it is available for a reaction with a bare conjugate, which is not bound to the analyte contained in the sample.

Another aspect of the present invention provides a chromatographic strip which further contains a second ligand that does not react with the analyte and a labeling material that can generate a detectable signal and bind to the second ligand in a conjugate pad thereof and further includes, in a detection pad thereof, a control line through which the migration of a sample can be confirmed, wherein the second ligand and the labeling material are joined as a second conjugate before or during the migration of a sample, the second conjugate can migrate to the detection pad during the migration of the sample, a material not reacting with the analyte or with the first conjugate has been immobilized in the control line, and the material immobilized in the control line can react with the second conjugate which does not bind to the analyte.

Compared to a conventional chromatographic strip provided with the detection pad including only a test line containing an immobilized material the same as or similar to the analyte and a control line through which the migration of a sample can be confirmed, the chromatographic strip of the present invention has more test lines and can be used not only for a qualitative analysis but also for a quantitative analysis with high reliability by allowing the comparison of the intensity of test line signals among the test lines or with the intensity of a control line signal which can be maintained at a constant level.

In addition, since the chromatographic strip of the present invention employs the competitive analysis, it can be used for the highly reliable quantitative determination, as well as the qualitative determination, of an analyte which is difficult to determine by the sandwich analysis due to having a relatively small size or having only one ligand-binding site.

Furthermore, the chromatographic strip of the present invention eliminates the need to use a special analytic tool, since it can be used for a semi-quantitative analysis with high reliability implemented by visual inspection or a general sensor.

The term "chromatography" used herein refers to an analysis method employing a combination of the principle of immunoreaction based on an antigen-antibody reaction, the principle of ligand-receptor coupling, the principle of a nucleic acid binding to a complementary nucleic acid, the principle of chromatography (i.e., samples and reagents are moved through a medium by a mobile phase), and the like. Briefly, in a porous membrane to which the analyte to be detected or an analog thereof has been applied and immobilized in advance, a solution containing a sample is allowed to migrate from one end of the membrane toward the immobilized analyte or analyte analog, and the membrane is examined for a reaction with the analyte. Although the term "immunoreaction" refers to a reaction between an antigen and an antibody in the general sense, when used herein it has a broader meaning and encompasses, in addition to a reaction between an antigen and an antibody, any type of reaction between two entities that specifically recognize each other, including, but not limited to, the mutually specific binding between a receptor and a ligand and a reaction between an enzyme and a substrate.

The chromatographic analysis is accomplished by the migration of a mobile phase through a medium by capillary action, carrying a sample containing the analyte along therewith. Therefore, as a medium for such chromatographic migration, a strip may be prepared and used. Components of such a chromatographic strip and functions thereof will be specifically described below.

A labeling material may be used to facilitate the detection of the above-described various specific binding reactions, including an antigen-antibody reaction, by visual inspection or a sensor. In addition, in order to allow the labeling material to bind to the analyte or to allow the analyte to bind to the labeling material, the labeling material may be coupled with a ligand capable of specifically binding to the analyte for its use.

As used herein, the term "conjugate" refers to an entity formed by joining the labeling material and the ligand, and the conjugate includes the first conjugate in which the labeling material for generating a detectable signal and the first ligand are joined together.

In the present invention, the "first ligand" refers to a material capable of reacting with the analyte and specifically binding to the analyte.

The joining between the labeling material and the first ligand may be accomplished physically or chemically. That is, the labeling material and the first ligand may be joined by passive adsorption, or the labeling material may be modified so as to have a reactive group so that it can be covalently bonded to the first ligand, but the present invention is not limited thereto, and the joining between the labeling material and the first ligand may be accomplished by a method known to a person skilled in the art.

However, the labeling material and the first ligand may have been previously joined and thus be present in the form of a conjugate in the conjugate pad even before allowing a sample to migrate through the chromatographic strip, or they may be present separately (i.e., not joined) in the conjugate pad until they are joined as a conjugate by a migrating sample, or, alternatively, they may be present in the form of a conjugate in a solution which can be added to the conjugate pad prior to use of the conjugate pad. In any of the above-described cases, the labeling material and the first ligand can migrate, as a conjugate, to the detection pad during the migration of a sample because they have not been immobilized to the conjugate pad.

As used herein, the term "labeling material" refers to a material for generating a signal that can be detected by visual inspection or a sensor. Examples of a material that can be used as the labeling material include colloidal gold (i.e., gold particles), latex particles, colored fine polystyrene particles, an enzyme, a fluorescent dye, a conductive polymer, a luminescent material, and magnetic particles, but the present invention is not limited thereto. In addition, the labeling material may have the inherent ability to spontaneously generate the signal (e.g., luminescence), or may generate the signal upon being externally stimulated (e.g., fluorescence).

As used herein, the term "ligand" refers to a material that can specifically bind to its counterpart. For example, in the specific binding between an antibody and an antigen and in the specific binding between a ligand and a specific receptor, the relevant materials function as a ligand with respect to its counterpart. Nonlimiting examples of the ligand may include a protein, an antigen, an antibody, DNA, RNA, PNA, and an aptamer, and in the present invention, any material besides those listed above may be used as the ligand as long as it exhibits the characteristic as defined above. "Ligand" as used throughout this specification refers to any material that can specifically bind to its counterpart as defined above unless specified as being a ligand that specifically binds to a specific receptor.

In a preferred embodiment of the present invention, the labeling material and the first ligand may have been previously joined and thus may be present in the form of a conjugate in the conjugate pad. In this case, separate solutions containing each of the labeling material and the first ligand at a known concentration may be prepared, mixed, and reacted for a predetermined time to prepare a conjugate solution, and the conjugate solution may be applied to the conjugate pad to prepare a conjugate pad containing a conjugate. Alternatively, the prepared conjugate solution may be added to the conjugate pad by a user prior to use of the conjugate pad. In this case, the concentration of each solution and the mixing ratio of the solutions may be determined by considering the binding ratio between the labeling material and the ligand. When it is mentioned that a ligand binds to a labeling material, it may refer to a case in which one ligand molecule binds to one labeling material molecule, a case in which a plurality of ligand molecules bind to one labeling material molecule, or a case in which a ligand molecule binds to a plurality of labeling material molecules. Although the specific binding ratio is not critical, it may be desirable that the ratio remains constant. The binding ratio may vary depending on the types of the labeling material and the ligand, and may be predicted by considering their relative molecular sizes, number of binding sites, and the like. For example, when a latex bead of several micrometers in size and an antibody are respectively used as the labeling material and the ligand, a plurality of the antibody molecules may bind to one latex bead. Although it may be preferable that the concentrations and mixing ratio of the antibody solution and the latex solution are adjusted such that the surface of each latex bead is saturated with the antibody molecules bound thereto and thus includes a uniform number of antibody molecules, the present invention is not limited thereto.

It has been described above that a conjugate solution of a known concentration can be prepared by mixing and reacting labeling-material and first-ligand solutions of known concentrations. Here, the conjugate solution is in the form of a liquid dispersion in which conjugate molecules of a predetermined concentration are uniformly dispersed, forming a solution.

The chromatographic strip is a chromatographic strip which has two or more test lines containing a predetermined amount of an immobilized material the same as or similar to the analyte, and may include: a conjugate pad containing a first ligand for reacting with the analyte contained in a sample or with a test line and a labeling material capable of generating a detectable signal and binding to the first ligand; and a detection pad having two or more test lines and a control line which are separated from one another.

In order to determine the analyte contained in a sample not only qualitatively but also quantitatively, two or more test lines have been immobilized to the detection pad to form "multiple competition assay test lines." In the detection pad of a chromatographic strip of the present invention, the control line may contain an additional material immobilized therein, in which case the material is either a material that is reactive with the first ligand of a bare conjugate consisting of a labeling material and a first ligand and carried by a migrating mobile phase through the detection pad but non-reactive with the analyte or a material that is non-reactive with both the analyte and the first ligand but reactive with the second ligand of a conjugate consisting of a labeling material and a second ligand.

In the present invention, the first ligand refers to a ligand which reacts with the analyte in a sample or with the analyte or analyte analog in the two or more test lines immobilized to the detection pad.

In the present invention, the second ligand refers to a ligand which reacts only with a material immobilized in the control line.

The second ligand is used in a specific amount determined to adjust the signal intensity. Since the detection of the analyte is accomplished by comparing the above-described signal intensity with the intensity of a test line signal which is dependent on the analyte concentration of a sample, the second ligand serves to control the range of detectable concentration of the analyte, and further, can contribute to the realization of a quantitative analysis with high reliability.

The two or more test lines of the chromatographic strip of the present invention contain a predetermined amount of the analyte or analyte analog that has been immobilized in the test lines and is reactive with the first ligand of the conjugate pad, and the amount of the immobilized material may be the same or different for each of the two or more test lines. Since the first ligand provided at the conjugate pad can specifically bind to the analyte, when a sample being analyzed does not contain the analyte, a conjugate consisting of the first ligand and a labeling material migrates along with the mobile phase and binds to the analyte (or analyte analog) immobilized in a test line of the detection pad to form a complex consisting of the analyte, the first ligand, and the labeling material, and the test line produces a strong signal. When a sample being analyzed contains the analyte, a complex consisting of the analyte of the sample, the first ligand, and the labeling material is formed and migrates to the detection pad along with the mobile phase, in which case as a larger amount of the labeling material and the first ligand is involved in forming a complex with the analyte, a smaller amount of the materials forms a complex with the analyte or analyte analog in a test line, and therefore, the test line produces a signal of lower intensity. That is, the decrease in the intensity of a test line signal is proportional to the amount of analyte in a sample.

The second ligand, which is further provided at the conjugate pad, is non-reactive with the analyte or with the first ligand and reacts only with (and binds to) a material immobilized in the control line of the detection pad to produce a signal.

When the signal intensity of each of the two or more test lines, which is dependent on the analyte concentration of a sample, is compared with the signal intensity of the control line and the ratio of signal intensity of each test line to the signal intensity of the control line is determined, a quantitative analysis effective over a wide range of analyte concentration can be realized.

It has been described above that the chromatographic analysis employs the principle of chromatography in which a mobile phase migrates through a medium, carrying the analyte therewith. Therefore, an analysis using a chromatographic strip requires a mobile phase for moving a sample containing the analyte along the strip. Accordingly, the assay kit of the present invention may further include a buffer solution. The buffer solution not only functions as a mobile phase for moving a sample along the chromatographic strip but also serves as a solvent for dissolving a conjugate, and, if necessary, may also serve as a diluent for diluting the sample. In addition, in the case of a whole-blood assay, the buffer solution may further include a component for inducing the lysis of the blood cell components such as red blood cells. A conventional buffer solution, such as phosphate-buffered saline (PBS) having a concentration of 10 mM to 1 M, a non-ionic or amphoteric surfactant, or a mixture thereof, may be used as the buffer solution without limitation, and the composition of the buffer solution and the amount of the buffer solution to be used may be appropriately selected according to the type of the desired reaction (e.g., an antigen-antibody reaction).

The conjugate pad is a pad containing a first predetermined amount of the above-described first ligand for reacting with the analyte and a labeling material capable of generating a detectable signal and binding to the first ligand. Here, the first ligand and the labeling material may have been previously joined and thus may be present in the form of a conjugate in the conjugate pad even before the migration of a sample, or they may be present separately (i.e., not joined) in the conjugate pad until they are joined as a conjugate by a migrating sample, or, alternatively, they may be present in the form of a conjugate in a solution which can be added to the conjugate pad prior to use of the conjugate pad.

In the present invention, the amount of the first ligand present in the conjugate pad can be adjusted to a predetermined specific amount. That is, the above-described first predetermined amount refers to the specific amount of the first ligand present in the conjugate pad. Since it has been decided that the conjugate pad contains a first predetermined amount of the first ligand, an inverse relationship may be observed between the number of complexes formed by the binding of the first ligand to the analyte and the number of bare conjugates not bound to the analyte, wherein the former increases and the latter decreases with an increase in the analyte concentration of a sample.

Preferably, the first ligand and the labeling material have been previously joined and thus are present in the form of a conjugate in the conjugate pad. Here, the conjugate pad may have been prepared by the above-described method of applying a conjugate solution of a known concentration to a pad.

Furthermore, the conjugate pad may further contain, as an internal control, a second conjugate consisting of a second ligand and a labeling material which are bound to each other. The second ligand refers to a ligand capable of specifically or non-specifically reacting with the material of the control line. The binding of the second conjugate to the material of the control line allows the sample migration to be recognized. A more detailed description of the control line will be provided below.

The detection pad is a medium through which the mobile phase and the sample migrate, wherein the migration of the mobile phase and the sample is driven by capillary action in the porous membrane constituting the detection pad. In addition, the detection pad has the above-described test lines and control line, which are separated from one another. One end of the detection pad may be connected to the conjugate pad, and the other end may be connected to an absorbance pad for providing a driving force for sample transport. The conjugate pad and the absorbance pad may be disposed such that they are partially overlapped with the detection pad. The detection pad may be made of a porous membrane, wherein examples of a usable porous membrane include a nitrocellulose membrane, a glass-fiber membrane, a polyethersulfone (PES) membrane, a cellulose membrane, a nylon membrane, and a combination thereof. Although a nitrocellulose membrane having a pore size of 5 µm to 15 µm is preferred as the porous membrane, the present invention is not limited thereto.

The principle of qualitative and quantitative analyses using the chromatographic strip of the present invention will be described in more detail.

In a conventional chromatographic strip utilizing a competition reaction, a test line has been formed in the detection pad by immobilizing a material the same as or similar to the analyte, and in the absence of the analyte in the mobile phase migrating and carrying the sample, the conjugate consisting of the first ligand and the labeling material is captured by binding to the analyte in the test line, which results in the production of a signal. When a sample being analyzed contains the analyte, the first ligand specifically reacts with the analyte to form a complex consisting of the analyte, the first ligand, and the labeling material, which migrates through the mobile phase, and the amount of the first ligand and the labeling material forming a complex with the analyte in the test line decreases as much as the amount of the complex consisting of the analyte, the first ligand, and the labeling material in the mobile phase has increased (i.e., a competition reaction), and accordingly, the signal intensity decreases with an increase in the analyte concentration of the sample. In this case, such a competition reaction takes place only when the concentration of the analyte present in the sample is sufficiently high, since when the analyte concentration of the sample being analyzed is low, in the presence of only one available test line, the formation of a complex between the analyte in the test line and the conjugate consisting of the first ligand and the labeling material is not affected by the binding between the first ligand and the analyte contained in the sample because the first ligand has a strong tendency to specifically bind to the analyte in the test line, and therefore, the change in test line color is almost unrecognizable by the naked eye or a sensor. Since the conventional chromatographic strip has low sensitivity and a narrow dynamic range, there are significant limitations in using it for the quantitative determination of the analyte.

However, unlike a typical chromatographic strip utilizing a competition reaction which has only one available test line, the chromatographic strip of the present invention has two or more test lines in which the analyte or an analyte analog has been immobilized and a competition reaction takes place so that even a sample of low analyte concentration can be analyzed (i.e., sensitivity is improved), and has been designed to implement a quantitative analysis which is effective over a wide concentration range. When three test lines are used and numbered according to their positions with respect to the conjugate pad (from closest to farthest) as shown in FIG. 2, the signal intensity decreases in the order of the test line 1 (T1) (i.e., the first of the test lines that the mobile phase carrying a sample encounters), where the signal intensity is strongest, the test line 2 (T2), and then the test line 3 (T3), so that separate quantitative graphs for the test line 1, the test line 2, and the test line 3 illustrating competition reactions can be obtained as a function of the analyte concentration of the sample. Therefore, it is possible to select an appropriate one among the graphs of the test lines 1, 2, and 3 in accordance with the concentration range of the analyte in a sample, in which case the graphs of the test lines 3, 2, and 1 are used for low, medium, and high concentrations, respectively, and as a result, the sensitivity to the analyte is improved, and the dynamic range is broadened from a low concentration to a high concentration. Since a solution for enabling the quantitative determination of the analyte concentration using a competition reaction between the analyte contained in the sample and a material immobilized in a test line of the detection pad, which is a material the same as or similar to the analyte, for a first predetermined amount of the first ligand of the conjugate pad has been devised and realized as a single strip, the present invention provides improved speed, improved ease of use, and in particular, improved quantitative analytical capabilities compared to conventional chromatographic analysis methods.

Specifically, when a liquid sample is introduced into the sample pad of the strip of the present invention, the mobile phase and the sample begin to migrate. In the conjugate pad, a first predetermined amount of the first ligand reacts, in a specific manner, with the analyte contained in the sample. A part of the first predetermined amount of the first ligand reacts with the analyte in the sample to form a complex consisting of the analyte, the first ligand, and the labeling material coupled with the first ligand as a conjugate, whereas the remainder of the first ligand which has not reacted with the analyte migrates, as a bare conjugate, along with the mobile phase. In this case, since the amount of the first ligand is fixed (i.e., the first predetermined amount), there is a tendency for the formation of the above-described complex to increase, whereas the number of the above-described bare conjugates decreases, as the analyte concentration of the sample increases.

The bare conjugates which contain the first ligand and the labeling material and have not been bound to the analyte contained in the sample are captured, while migrating, by the multiple test lines containing the analyte or an analyte analog immobilized therein, in which case the test lines provided at different locations produce signals of different intensities. In addition, the complexes consisting of the first ligand, the labeling material, and the analyte are not captured by the test lines while migrating but further migrate along with the mobile phase. The bare conjugates are captured by the test lines and thus produce a signal, and the complexes consisting of the first ligand, the labeling material, and the analyte are not captured and thus do not produce a signal.

Since both the complex and the bare conjugate contain a labeling material, it is possible to determine the presence/absence of the analyte in a sample and/or the quantity of the analyte by measuring signals which the labeling material of the bare conjugate captured by the test lines generates at the test lines.

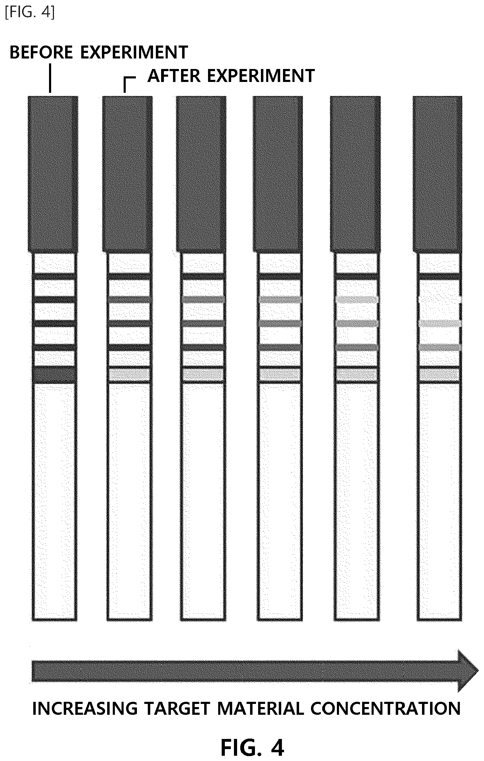

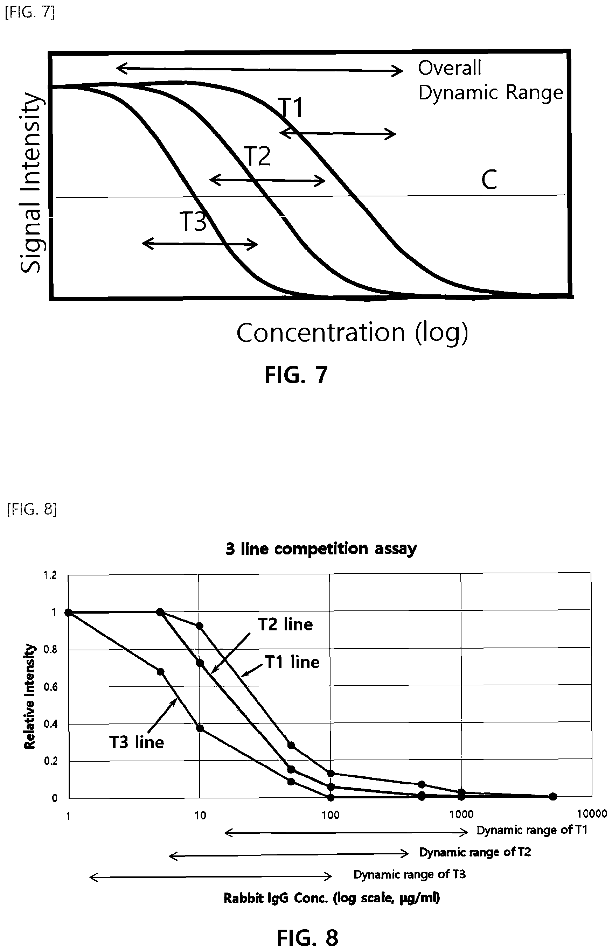

It has been described above that the number of bare conjugates decreases and the number of complexes increases with an increase in the analyte concentration of the sample. Therefore, there is a tendency for the signal intensity of the test lines capturing the bare conjugate to decrease as the analyte concentration of the sample increases. In addition, since multiple test lines are used as in the exemplary case as shown in FIG. 4, in which three test lines and one control line are used and the test lines are numbered according to their positions with respect to the conjugate pad (from closest to farthest), the test line 1 (i.e., the first of the test lines that the mobile phase carrying a sample encounters) produces the strongest signal due to the binding of the bare conjugate to the analyte therein and is the last of the test lines to be affected (i.e., to show a decrease in signal intensity) by the amount of analyte in a sample. Next, in the case of the test line 2, the amount of the bare conjugate migrating along with the mobile phase has been reduced due to a reaction with the test line 1, and compared to the test line 1, a decrease in the signal intensity of the test line 2 is observed at a lower analyte concentration of a sample. In the case of the test line 3, the amount of the bare conjugate migrating along with the mobile phase has been even more reduced due to a reaction with the test lines 1 and 2, and compared to the test line 2, a decrease in the signal intensity of the test line 3 is observed at a lower analyte concentration of a sample. Accordingly, separate quantitative graphs for the test line 1, the test line 2, and the test line 3, which illustrate competition reactions, can be obtained as a function of the analyte concentration of a sample as shown in FIG. 7. Since the graphs for the test lines 3, 2, and 1 can be respectively used for low (T3), medium (T2), and high (T1) concentration ranges as shown in FIG. 7, it is possible to select an appropriate quantitative graph for carrying out quantification with one experiment at a given concentration range of analyte in a sample, and therefore, it is possible to improve the sensitivity to the analyte and broaden the overall dynamic range (T1-T3) from a low concentration to a high concentration.

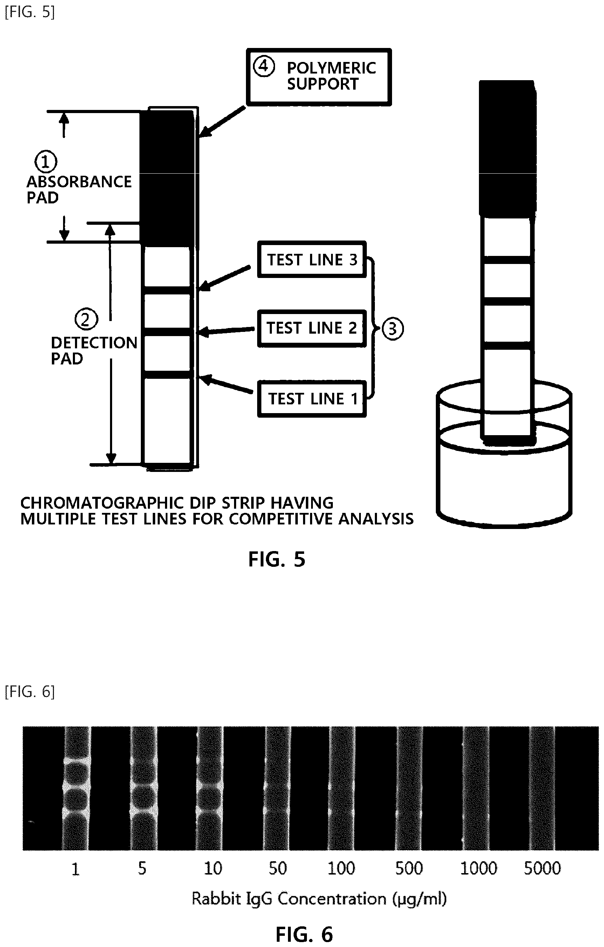

More specifically, as shown in FIG. 7, since the signal intensity of the test line 1, which is maintained constant to the point where the analyte concentration of a sample is M1 (corresponding to a lower concentration), is gradually decreased with an increasing concentration at higher concentrations until it finally converges to zero, the signal intensity of the test line 2, which is maintained constant to the point where the analyte concentration of the sample is M2 (corresponding to a lower concentration), is gradually decreased with an increasing concentration at higher concentrations until it finally converges to zero, and the signal intensity of the test line 3, which is maintained constant to the point where the analyte concentration of the sample is M3 (corresponding to a low concentration), is gradually decreased with an increasing concentration at higher concentrations until it finally converges to zero, there is a dynamic range (T1, T2, and T3, respectively) for each one of the test line 1, the test line 2, and the test line 3, and it is expected that the overall dynamic range (T1∼TN) for a total of N test lines will be broadened to lower concentrations as the number of the test lines increases. With regard to the test lines, it has been described above that there is a tendency for the signal intensity to decrease as the amount of the analyte increases. FIGS. 4 and 6 illustrate how the signal intensity of multiple test lines of the present invention changes as the concentration of the analyte increases.

As a result, it is possible to qualitatively and quantitatively determine the analyte using a signal from the labeling material bound to the first ligand captured by the two or more test lines at which the competition reactions occur. More specifically, it is possible to determine the concentration of the analyte by comparing test line signals with the signal data reference completed in advance based on known analyte concentrations. Here, the concentration of the analyte may be determined semi-quantitatively by determining the signal intensity by visual inspection, or it may be determined more precisely through quantitative analysis using a reader such as a densitometer.

In the present invention, the detection pad may further have a control line through which the migration of a sample can be confirmed.

As used herein, the term "control line" refers to a portion that produces a signal of constant intensity irrespective of the sample or of the analyte concentration of the sample. This invariant control line may be formed by a method similar to the above-described method of forming a test line, except that when forming the control line, the material that is immobilized may be a material which does not bind to the material of interest (i.e., analyte) but specifically or non-specifically binds to the second ligand of the second conjugate and thus can capture the second conjugate migrating through the detection pad along with the sample due to the mobile phase. Alternatively, the control line may be formed by immobilizing a material which specifically or non-specifically binds to, and thus can capture, an additional material which is moved through the detection pad by the mobile phase along with the sample and either is a labeling material itself or is bound to a labeling material. In other words, the control line is formed by immobilizing a material capable of producing a signal of constant intensity irrespective of the analyte concentration of the sample or the presence or absence of the analyte in the sample. Examples of a material that can be used in the control line include anti-rabbit IgG antibodies, anti-chicken antibodies, anti-IgY antibodies, streptavidin, bovine serum albumin, and the like.

Since the control line containing a material of a fixed concentration immobilized therein always generates a signal of constant intensity, the signal intensity of the control line can be used as a reference to which the signal intensity of the N test lines is compared. Further, the signal intensity of each test line may be expressed as a ratio with respect to the signal intensity of the control line so that it can be more easily recognized, and it is possible to correlate the level of analyte concentration with the signal intensity of each test line. In a quantitative analysis, the signal intensity of the control line can be used as a reference to which the signal intensity of a test line is compared. That is, the ratio of the signal intensity of each test line to the signal intensity of the control line is the signal data that can be used as the internal reference.

For example, when three test lines have been provided as shown in FIG. 7 and it has been confirmed, using the analyte of a known concentration, that the test lines 1, 2, and 3 respectively produce a signal of 10-, 5-, and 3-fold the signal intensity of the control line for the analyte concentration of 10 ng/ml and a signal of 5-, 3-, and 1-fold the signal intensity of the control line for the analyte concentration of 30 ng/ml, the signals from the test lines 1, 2, and 3 which are respectively 7-, 4-, and 2-fold the signal intensity of the control line for a unknown analyte concentration indicate that the analyte concentration is between 10 ng/ml and 30 ng/ml. Since the signal intensity of the test lines is compared with the signal intensity of the control line, it is easy to determine the concentration not only by an easy-to-use reader such as a POCT medical device but also by the naked eye.

The position and number of the test lines utilizing multiple competition reactions and the position of the control line may be appropriately selected according to an antigen-antibody reaction of interest or the like, but the present invention is not limited thereto. The generation of the control line signal indicates the successful migration of a sample, and the quantitative determination of the analyte is enabled by the comparison of the intensity of test line signals and control line signals with respect to the signal data reference completed in advance. A description of the signal data reference will be provided below.

In the present invention, in the case of a quantitative analysis conducted based on the signals from the immobilized multiple test lines, which are of different intensities for different test lines at a given concentration, and the signal intensity of the control line, the dynamic range for the analyte concentration of a sample may be from 0.1 ng/ml to 1 mg/ml. Since the chromatographic strip of the present invention has multiple test lines and different test lines produce signals of different intensities at a given concentration, both a test line appropriate for a low concentration situation which could not be dealt with only one test line where a competition reaction for the analyte occurs and a test line appropriate for a high concentration situation are available for use, and therefore, the single strip of the present invention having a combination of the multiple test lines is characterized in that it enables a quantitative analysis which the conventional test line was inadequate for, has a broadened dynamic range for concentration, and exhibits improved sensitivity.

The configuration of the chromatographic strip of the present invention will be described in more detail.

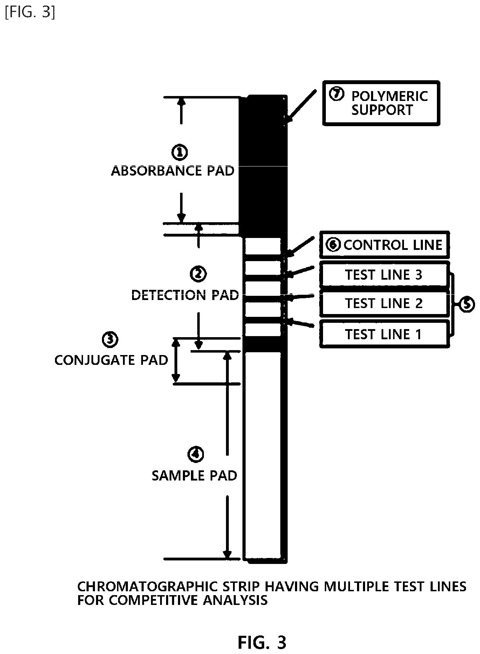

The chromatographic strip may include: a sample pad into which a sample to be analyzed for the presence or absence of the analyte is introduced; a conjugate pad, one end of which is connected to the sample pad; a detection pad, one end of which is connected to the other end of the conjugate pad; an absorbance pad, one end of which is connected to the other end of the detection pad and which provides a driving force for transporting the sample from the sample pad; and a solid support provided at the bottom portion of the chromatographic strip. The configuration of the chromatographic strip of the present invention is illustrated in FIG. 3.

The solid support may be formed of a material selected from the group consisting of nitrocellulose, nylon, polyvinylidene fluoride (PVDF), glass, and other polymeric materials. The attachment of the solid support to the upper portions of the strip can improve the durability and ease of handling and storage of the prepared strip, and further, allows easy mounting of the strip inside a case. Examples of a polymeric material that can be used for the solid support include a polypropylene film, a polyester film, a polycarbonate film, an acrylic film, and the like, but the present invention is not limited thereto.

Still another aspect of the present invention provides, as a diagnostic kit, a kit in which the chromatographic strip is further fixed within a case, wherein a lower portion of the case includes a guide and a strip supporting portion, and an upper portion of the case includes a sample inlet and, at the position corresponding to multiple test lines and a control line, a result display window.

The chromatographic strip may be further fixed within a case. The inside of a lower portion of the case may include: multiple guides for placing the chromatographic strip at a suitable position and fixing or compressing the chromatographic strip; and/or a strip supporting portion. Optionally, an upper portion of the case also may include guides and a strip supporting portion which are positioned so as to coincide with the positions of the guides and strip supporting portion of the lower portion of the case. That is, the guides and/or the strip supporting portion may be provided only at the lower portion of the case or at both the upper and lower portions of the case as needed. In addition, the upper portion of the case may include a sample inlet and, at the position corresponding to the test lines and the control line, a result display window for detecting a signal generated by the labeling material. The sample inlet may be in the form of a hole, a slit, or the like, and may be provided at a position which is on one end of the detection pad on the opposite side of the absorbance pad relative to the test lines and, at the same time, is spaced sufficiently apart from the test lines in order to allow a sample to migrate along the membrane. The result display window may be provided above the detection pad, and may be large enough to allow all the multiple test lines and the control line to be recognized from the outside by visual inspection or a sensor. There is no limitation on the size and shape of the result display window as long as the multiple test lines and the control line can be viewed therethrough.

The upper and lower portions of the case may be produced using a common polymeric material such as polycarbonate and acrylonitrile butadiene styrene (ABS), but the present invention is not limited thereto. The upper and lower portions of the case may be produced as separate units which can be coupled by conventional means due to having coupling grooves, coupling protrusions, and the like, or may be produced as one integrated unit in some cases.

Further, in the diagnostic kit, there may be provided an accompanying signal data reference including a color reference table for the multiple test lines and the control line which has been obtained for samples of various known analyte concentrations.

In the present invention, the signal data reference refers to the signal intensity data which has been obtained from the multiple test lines and the control line of the chromatographic strip of the present invention for samples of various known analyte concentrations and arranged. Such signal data reference can be arranged in various ways, typically in terms of the analyte concentration and the corresponding colors of the multiple test lines and the control line. For example, there is a similar case in which a litmus test strip for measuring pH is provided with an accompanying color reference table listing various pH values and corresponding colors of the test strip. In particular, the above-described signal data reference including a color reference table allows a semi-quantitative analysis to be implemented easily and quickly because the intensity of signals for the analyte can be easily compared with the signal data reference by simple visual inspection. In this regard, the above-described diagnostic kit allows a qualitative analysis or a semi-quantitative analysis to be implemented through the visual inspection of the multiple test lines and the control line for the presence and intensity of signals generated by the labeling material. That is, it is possible to determine the presence or absence of the analyte in a sample and the concentration of the same, by allowing the sample to migrate through the kit and then determining the presence or absence of signals appearing on the plurality of test lines and the control line and comparing the intensity of signals with respect to the signal data reference previously described as being capable of having an accompanying color reference table.

Yet another aspect of the present invention provides, as a method of qualitatively or quantitatively determining an analyte in a sample using the above-described chromatographic strip, an analysis method including: a first step of introducing a sample into the conjugate pad or a pad preceding the conjugate pad and allowing the sample to migrate; a second step of identifying the presence or absence of a signal generated by the labeling material in the multiple test lines and the control line and determining the intensity of such signals; and a third step of determining the amount of the analyte by comparing the intensity of the signals detected from the multiple test lines and the control line with respect to a signal data reference, wherein the signal data reference has been obtained by carrying out the first and second steps with respect to samples of various known analyte concentrations.

Details of the principle of the method of qualitatively and/or quantitatively determining an analyte in a sample using the chromatographic strip of the present invention have been described above. In addition, a description of the signal data reference has been provided above.

Specifically, the above-described analysis method may be carried out in the following order. First, a liquid sample containing the material of interest (i.e., analyte) may be introduced into the conjugate pad or a pad preceding the conjugate pad. That is, the liquid sample can be introduced into the strip by way of injecting it directly into the conjugate pad but is preferably introduced through a pad preceding the conjugate pad, which is, for example, the sample pad. In addition, the sample may be uniformly mixed with an additional buffer solution such as PBS and introduced, as a mixture, into the strip in the above-described manner.

Once loaded (introduced) into the chromatographic strip as described above, the sample begins to migrate, and the success of sample migration can be confirmed later through the control line. When the sample migration has been successful, then the multiple test lines and the control line are inspected for the presence of signals generated by the labeling material, and the signal intensity is measured. When the multiple test lines exhibit a change (i.e., a signal), this indicates that the analyte is present within the sample (i.e., qualitative analysis). Furthermore, when the intensity of signals detected from the multiple test lines and the control line is compared with respect to the signal data reference, it is possible to estimate the concentration at which the analyte is contained in the sample (i.e., quantitative analysis).

In a specific exemplary embodiment of the present invention, an analysis for rabbit immunoglobulin G (rabbit IgG; analyte) was carried out using a chromatographic dip strip having an additional control line, and it was confirmed that a highly reliable quantitative analysis sensitive to a wide range of analyte concentrations, from 1 µg/ml to 5,000 µg/ml, can be implemented using a fluorescent reader (FIG. 6).

Examples of a sample which can be subjected to the analysis of the present invention include all types of biological samples, such as whole blood, blood cells, blood serum, blood plasma, bone marrow fluid, sweat, urine, tears, saliva, skin, mucous membrane, and hair separated from a mammal which is preferably a human, as well as general aqueous solutions, and for example, the sample is blood. Here, the blood used as the sample may be the plasma component or the serum component which excludes the blood cell components. When whole blood is used, a component capable of inducing the lysis of the blood cell components may be added to a buffer solution being used. However, this is only one example of a sample which can be subjected to the analysis of the present invention, and the present invention is not limited to a particular type of sample.

The analysis method of the present invention can be useful for a diagnosis based on a semi-quantitative or quantitative analysis for a protein having a small size (e.g., a peptide) or only one available binding site, vitamin D, hIgG, hIgE, an allergen, an antibiotic, or other various drugs.

### [Advantageous Effects]

By having multiple test lines utilizing measurable competition reactions, the chromatographic strip of the present invention provides the following effects: the difficulty of analyte quantification can be overcome; a quantitative analysis with high reliability, as well as a qualitative analysis, can be implemented quickly and conveniently using a fluorescent reader; furthermore, since multiple test lines are used, improved sensitivity to an analyte and an improved dynamic range for the analyte concentration are attained.

### [Description of Drawings]

FIG. 1 is a cross-sectional overview of a conventional assay strip used for chromatographic analysis.
FIG. 2 is a cross-sectional overview of an assay strip used for the chromatographic analysis of the present invention.
FIG. 3 is a schematic diagram of a chromatographic strip according to an overview of the present invention.
FIG. 4 is an overview illustrating how the multiple test lines and the control line of a chromatographic strip of the present invention appear before and after the introduction of a sample.
FIG. 5 is a schematic diagram illustrating a method using a dip strip according to one exemplary embodiment of the present invention.
FIG. 6 is an image comparing the results of analyzing samples of various analyte concentrations according to one exemplary embodiment of the present invention. Here, rabbit IgG was used as the analyte.
FIG. 7 is a quantitative graph derived by multiple test lines according to an overview of the present invention.
FIG. 8 is a quantitative graph based on the results of an experiment of the present invention, in which the signal intensity of each one of multiple test lines is plotted as a function of the analyte concentration and the dynamic range is indicated.

### [Best Mode]

Hereinafter, the present invention will be described in more detail with reference to exemplary embodiments. These embodiments are provided to explain the present invention more specifically, and the scope of the invention is not limited to the embodiments. The embodiments utilized a dip-strip method, which is one of the various chromatographic analysis methods.

### Example 1: Preparation of chromatographic dip strip

### A. Preparation of detection pad having test lines

Three test lines were formed on a nitrocellulose membrane. The nitrocellulose membrane was laminated onto a polymeric card using a laminator. Subsequently, test lines 1, 2, and 3 spaced apart from one another at regular intervals were formed by applying, as the analyte or an analyte analog, rabbit IgG (Arista Biologicals Inc., USA) to corresponding positions using an automatic dispenser, and dried at a temperature of 25 °C to 30 °C for two days (i.e., 48 hours).

### B. Preparation of absorbance pad

The absorbance pad, the moisture of which had been completely removed in a dryer, was used as it was without any further treatment, and was cut to an appropriate size prior to its use.

### C. Fabrication of chromatographic strip

The detection pad and the absorbance pad prepared through each of the above-described processes were assembled according to the configuration illustrated in FIG. 5.

That is, the detection pad was attached to an adhesive applied to a polymeric support, and the absorbance pad was attached such that one end of the detection pad was overlapped with one end of the absorbance pad. About 2±1.0 mm of the resultant was cut out using a cutter, and thereby a strip as shown in FIG. 3 was obtained.

In FIG. 5, each reference numeral denotes the following:
1: Absorbance pad; 18±4 mm×4±2 mm
2: Nitrocellulose detection pad; 25±5 mm×4±2 mm
3: Test lines 1, 2, and 3 containing immobilized rabbit IgG
4: Polymeric support

### Example 2: Analysis using chromatographic dip strip

One hundred microliters of a sample solution containing rabbit IgG as the analyte and PBS was dispensed into each well of a 96-well plate. The sample solutions were prepared so as to contain the rabbit IgG at concentrations of 0, 5, 10, 50, 100, 500, 1000, and 5000 µg/ml. Subsequently, a first predetermined amount of a solution containing a conjugate consisting of a labeling material (fluorescent material) and a first ligand (anti-rabbit IgG) was added to the sample solutions.

Then, the chromatographic dip strips fabricated in Example 1 were dipped into the sample solutions contained in the 96-well plate to allow sample migration for 10 minutes.

FIG. 6 is an image showing the result of migration in chromatographic strips at various rabbit IgG concentrations.

It was confirmed that the signal intensity of the test lines 1, 2, and 3 varies with the analyte concentration and that a different quantitative curve for the analyte concentration can be obtained for each test line.

In addition, it was confirmed that the chromatographic strip exhibited a wide dynamic range for the analyte concentration, from 5 µg/ml or less to 5,000 µg/ml or more.

## Claims

1. A chromatographic strip comprising a conjugate pad and a detection pad, wherein:
the conjugate pad contains a first ligand which has the ability to react with a target material and a labeling material which has the ability to generate a detectable signal and bind to the first ligand, wherein the first ligand and the labeling material are joined together as a first conjugate before or during sample migration, and the first conjugate has the ability to migrate to the detection pad during the sample migration; and
the detection pad has two or more test lines which are separated from each other and, by containing an immobilized material the same as or similar to the target material, have the ability to react with a bare conjugate which is not bound to the target material present in the sample.

2. The chromatographic strip of claim 1, wherein:
the conjugate pad contains a predetermined amount of the first ligand, a part of which reacts with the target material in the sample and forms a complex in which the target material is bound to the first conjugate including the first ligand and the labeling material; and
as the concentration of the target material in the sample increases, the number of the formed complexes increases, whereas the number of the bare conjugates decreases.

3. The chromatographic strip of claim 2, wherein:
the complex including the first ligand, the labeling material, and the target material is not captured by the test line;
the bare conjugate, which does not bind to the target material present in the sample and includes the first ligand and the labeling material, is captured by the test line and produces a signal by reacting with a material, a predetermined amount of which has been immobilized in the test line and which is the same as or similar to the target material; and
the chromatographic strip allows the determination of the presence and/or quantity of the target material in the sample based on the measurement of a signal generated by the labeling material in the test line.

4. The chromatographic strip of claim 3, wherein in all of the test lines immobilized to the detection pad, the intensity of a signal decreases as the concentration of the target material in the sample increases.

5. The chromatographic strip of claim 4, wherein in the test line, the intensity of a signal, which is maintained constant to a point where the concentration of the target material in the sample is M, is gradually decreased at higher concentrations until it finally converges to zero,
wherein the M is the maximum concentration of the target material in a sample which results in the saturation of the test line with the bare conjugate, wherein the saturation of the test line refers to a case in which all of the predetermined amount of the material which has been immobilized in the test line and is the same as or similar to the target material has reacted with the bare conjugate.

6. The chromatographic strip of claim 5, wherein, since the number of the test lines used has been increased to N (N≥2) so that a plurality of the test lines are used, the intensity of a signal, which is maintained constant to a point where the concentration of the target material is Mn, is gradually decreased at higher concentrations until it finally converges to zero.

7. The chromatographic strip of claim 1, wherein:
the conjugate pad contains a second ligand which does not react with the target material and a labeling material which has the ability to generate a detectable signal and bind to the second ligand, wherein the second ligand and the labeling material are joined together as a second conjugate before or during sample migration, and the second conjugate has the ability to migrate to the detection pad during the sample migration; and
the detection pad further has a control line configured to allow the confirmation of sample migration, wherein the control line contains an immobilized material which does not react with the target material or with the first conjugate and has the ability to react with the second conjugate, wherein the second conjugate does not bind to the target material.

8. The chromatographic strip of claim 1, which includes:
a sample pad into which a sample to be analyzed for the presence or absence of the target material is introduced;
the conjugate pad, one end of which is connected to the sample pad;
the detection pad, one end of which is connected to the other end of the conjugate pad;
an absorbance pad, one end of which is connected to the other end of the detection pad and which provides a driving force for transporting the sample from the sample pad; and
a solid-type support provided at the bottom portion of the chromatographic strip.

9. The chromatographic strip of claim 8, wherein the support is formed of a material selected from the group consisting of nitrocellulose, nylon, polyvinylidene fluoride (PVDF), glass, and other polymeric materials.

10. The chromatographic strip of claim 1, wherein the ligand is a protein, an antigen, an antibody, DNA, RNA, PNA, or an aptamer.

11. The chromatographic strip of claim 1, wherein the labeling material is colloidal gold, latex particles, colored fine polystyrene particles, an enzyme, a fluorescent dye, a conductive polymer, a luminescent material, or magnetic particles.

12. The chromatographic strip of claim 1, wherein the target material has only one available binding site for the ligand.

13. The chromatographic strip of claim 1, wherein a dynamic range for target material concentration of a sample is from 1 ng/ml to 1 mg/ml.

14. A diagnostic kit comprising the chromatographic strip of any one of claims 1 to 13 and an additional case in which the chromatographic strip is fixed, the diagnostic kit including:
a lower portion which includes a guide and a strip supporting portion; and
an upper portion which includes a sample inlet and, at the position corresponding to the test lines and the control line, a result display window.

15. The diagnostic kit of claim 14, which is provided with an accompanying signal data reference including a color reference table for the test lines and the control line, wherein the color reference table has been obtained for samples of various known target material concentrations.

16. The diagnostic kit of claim 15, which allows a qualitative analysis or a semi-quantitative analysis to be implemented through the visual inspection of the test lines and the control line for the presence/absence and intensity of a signal generated by a labeling material.

17. The diagnostic kit of claim 15, which allows a quantitative analysis to be implemented through the determination of the presence/absence and intensity of a signal generated by a labeling material in the test lines and the control line using a medical device including a reader.

18. An analysis method of qualitatively or quantitatively determining a target material in a sample using the chromatographic strip of any one of claims 1 to 13, the method comprising:
a first step of introducing the sample into the conjugate pad or a pad preceding the conjugate pad and allowing the sample to migrate;
a second step of determining the presence/absence and intensity of a signal generated by a labeling material in the test lines and the control line; and
a third step of determining the amount of the target material by comparing the intensity of signals detected from the test lines and the control line with respect to a signal data reference, wherein the signal data reference has been obtained by carrying out the first step and the second step with respect to samples of various known target material concentrations.

19. The analysis method of claim 18, wherein in the second step, the determination of the presence/absence and intensity of a signal generated by a labeling material in the test lines and the control line is carried out using a densitometer.
